# EUROPÄISCHE PATENTSCHRIFT

(11) **EP 0 789 700 B1**
(45) Veröffentlichungstag und Bekanntmachung des Hinweises auf die Patenterteilung: **16.01.2002**
(21) Anmeldenummer: 95909741.1
(22) Anmeldetag: 18.02.1995
(51) Int. Cl.: C07D 495/04, A61K 31/415, C07F 3/02

(54) **VERBESSERTES VERFAHREN ZUR HERSTELLUNG EINES D-(+)-BIOTIN-ZWISCHENPRODUKTES**
IMPROVED PROCESS FOR MAKING A D-(+)-BIOTINE INTERMEDIATE PRODUCT
AMELIORATION APPORTEE A UN PROCEDE DE FABRICATION D'UN PRODUIT INTERMEDIAIRE DE LA D-(+)-BIOTINE

(30) Priorität: 30.03.1994 DE 4411101
(43) Veröffentlichungstag der Anmeldung: 20.08.1997
(73) Patentinhaber: MERCK PATENT GmbH, 64293 Darmstadt (DE)
(72) Erfinder: ECKSTEIN, Jürgen, D-64380 Rossdorf (DE); KOPPE, Thomas, D-64291 Darmstadt (DE); SCHWARZ, Michael, D-64521 Gro -Gerau (DE); CASUTT, Michael, D-64646 Heppenheim (DE)
(86) Internationale Anmeldenummer: EP9500593
(87) Internationale Veröffentlichungsnummer: WO9526965

(56) Entgegenhaltungen:
- EP-A- 0 154 225
- EP-A- 0 273 270
- EP-A- 0 502 392
- DE-A- 2 058 234
- CHEMICAL ABSTRACTS, vol. 106, no. 5, 2. Februar 1987, Columbus, Ohio, US; abstract no. 33049h, KOZO SHIMAKO ET AL 'Preparation of thiophene derivatives as intermediates in vitamin H synthesis' Seite 552 ; & JP,A,61 151 194 (SUMITOMO)
- CHEMICAL ABSTRACTS, vol. 107, no. 11, 14. September 1987, Columbus, Ohio, US; abstract no. 96510t, MASAYOSHI MINAMII 'Hydroxytetrahydrothiophene derivatives' Seite 671 ; & JP,A,62 048 686 (SUMITOMO)
- CHEMICAL ABSTRACTS, vol. 70, no. 5, 3. Februar 1969, Columbus, Ohio, US; abstract no. 19984r, ICHIRO ISAKA ET AL 'Biotin synthesis. IV. A new synthesis of biotin by Grignard reaction of 1,4-butylenedimagnesium kalide' Seite 1990 ; & YAKUGAKU ZASSHI, Bd.88, Nr.8, 1968 Seiten 964 - 970

## Beschreibung

Die vorliegende Erfindung betrifft ein verbessertes Verfahren zur Herstellung von heterocyclischen Verbindungen, welche als Zwischenprodukte für die Herstellung von D-(+)-Biotin geeignet sind, sowie ein Verfahren zur Herstellung von D-(+)-Biotin selbst.

D-(+)-Biotin ist eine seit langem bekannte Substanz, und es sind demgemäß auch bereits eine Reihe Verfahren zu deren Herstellung bekannt. Den technisch interessanten Verfahren ist die Notwendigkeit gemeinsam, auf irgendeiner Stufe die Carboxybutylseitenkette an das Ringsystem anzuhängen. Hierfür sind verschiedene Lösungen bekannt, wie etwa der Aufbau der Seitenkette nach dem Verknüpfungsschema C₄ + C₁ → C₅ oder auch C₃ + (C₃-C₁ = C₂) → C₅ (z.B. CH-PS 556 867).

Es ist auch bekannt, die Seitenkette in einer Stufe mittels einer Wittig-Reaktion mit dem Ringsystem zu verknüpfen (z.B. EP-OS 0 084 337). Alle diese Verfahren haben jedoch den Nachteil, daß sie entweder über ein verhältnismäßig große Anzahl von Reaktionsstufen verlaufen oder aber einen relativ großen Aufwand zur Isolierung des gewünschten Endproduktes erfordern.

Weiterhin ist aus der EP-OS 0 154 225 die Einführung der Seitenkette durch Umsetzung des Thiolactons der Formel I mit 4-(2.4.10-Trioxaadamantyl)-butylmagnesiumbromid bekannt.

Daneben ist die Einführung der Carboxybutylseitenkette über eine "Bis-Grignard-Reaktion" mit einem 1,4-Dihalogenmagnesiumbutan und anschließender Carboxylierung mit Kohlendioxid seit langem bekannt. Nach der JP-B-003,580/71 und der DE-OS 20 58 234 wird die Reaktion in einem Lösungsmittelgemisch aus Diethylether und Toluol durchgeführt.

Dagegen schlagen die JP-A-280,037/84 und die EP-A 0 273 270 vor, die Reaktion in Tetrahydrofuran in Gegenwart von Tetramethylethylendiamin (TMEDA) durchzuführen.

In allen diesen Fällen wird das Additionsprodukt der Formel III 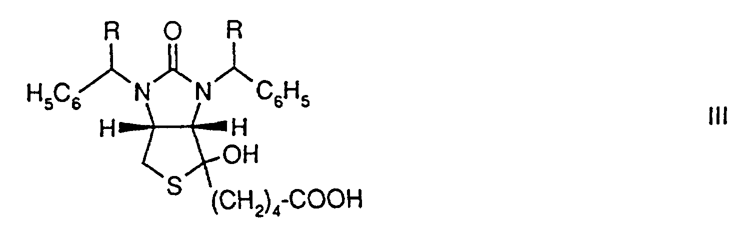 zuerst zumindestens als Rohextrakt isoliert und dann dehydratisiert.

Diese Verfahren weisen jedoch verschiedene Nachteile auf, welche sie für eine technische Durchführung als ungeeignet erscheinen lassen.

Lediglich nach dem in der JP-A-280,037/84 beschriebenen Verfahren kann das D-(+)-Biotin-Zwischenprodukt der Formel I in einer sehr guten Ausbeute hergestellt werden.

Jedoch erfordert dies den Einsatz des als reizend eingestuften Cosolvens TMEDA, wodurch die Aufarbeitung des Reaktionsgemisches besonders aufwendig wird, da dieses Cosolvens nicht ins Abwasser gelangen darf.

Desweiteren wird das dort beschriebene Verfahren in hoher Verdünnung durchgeführt, und die Durchführung der Carboxylierung bei Temperaturen zwischen -40 und -45 °C ist im technischen Maßstab nur schwer zu verwirklichen.

Weiterhin wird die Dehydratisierung des Grignard-Adduktes der Formel III in einen Lösungsmittelgemisch aus Toluol/Tetrahydrofuran durchgeführt, welches nach der Aufarbeitung zur Recyclisierung in einem aufwendigen Prozeß getrennt werden muß.

Aufgabe der vorliegenden Erfindung war es nun, ein Verfahren aufzuzeigen, bei dem das D-(+)-Biotin-Zwischenprodukt der Formel I in THF als Lösungsmittel, ohne den Einsatz des Cosolvens TMEDA in einer verbesserten Raum-Zeit-Ausbeute hergestellt werden kann.

Es bestand somit ein Bedürfnis nach einem technisch einfachen Verfahren, gemäß welchem die Seitenkette in guter Ausbeute und möglichst in einem "Eintopf-Verfahren" an das Ringsystem angehängt werden kann. Dies ist nunmehr mittels des erfindungsgemäßen Verfahren möglich.

Gegenstand der Erfindung ist somit ein verbessertes Verfahren zur Herstellung eines D-(+)-Biotin-Zwischenproduktes der Formel I, 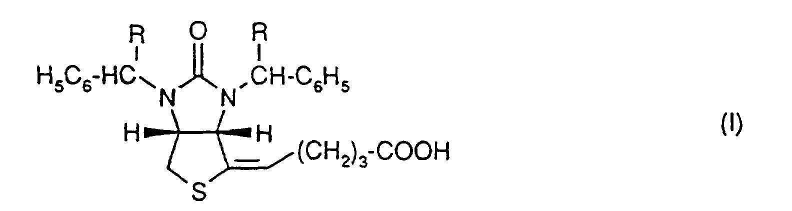 worin
- R: H oder C₁₋₆-Alkyl bedeutet,
durch Umsetzen des Thiolactons der Formel II, 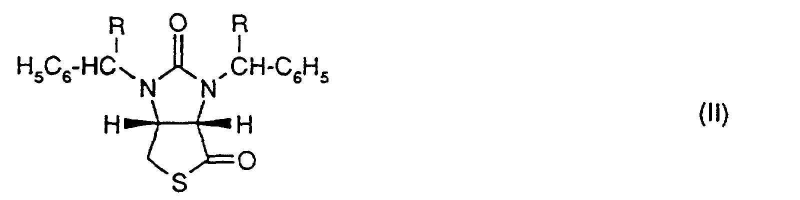 worin R die angegebene Bedeutung besitzt, mit 1,4-Dimagnesiumchlorbutan und Kohlendioxid in einem inerten Lösungsmittel und anschließende Wasserabspaltung mit einer Mineralsäure, dadurch gekennzeichnet, daß man nacheinander folgende Schritte durchführt:
a) Umsetzung von 1,4-Dichlorbutan mit Magnesium-Spänen in Gegenwart katalytischer Mengen von 1,2-Dibromethan in Tetrahydrofuran als Lösungsmittel,
b) Hinzufügen des Thiolactons der Formel II gelöst in Tetrahydrofuran bei Temperaturen zwischen -25° und -15 °C und gegebenenfalls Nachrühren,
c) Überleitung von CO₂ bei Temperaturen zwischen -25° und +15 °C,
d) Zugabe von Schwefelsäure, zur Neutralisierung und Dehydratisierung,
e) Separieren der Phasen und unter reduziertem Druck einengen,
f) Lösen des Rückstandes in einem Kohlenwasserstoff als Lösungsmittel und Waschen der organischen Phasen mit wäßriger Lauge,
g) Ansäuern der wäßrigen Phase mit einer Mineralsäure und Extraktion der wäßrigen Phase mit einem Kohlenwasserstoff,
h) Einengen der organischen Phase g) unter reduziertem Druck.

Bevorzugte Ausführungsformen des erfindungsgemäßen Verfahren sind:
a) Verfahren, wobei Schritt a) bei Temperaturen zwischen 40 und 70 °C durchführt.
b) Verfahren, wobei man in Schritt a) 1,6 bis 2,2 Mol Magnesium, 0,005 bis 0,020 Mol 1,2-Dibromethan, und insgesamt 1,5 bis 2,2 kg Tetrahydrofuran bezogen auf 1 Mol 1,4-Dichlorbutan, einsetzt.
c) Verfahren, wobei man in Schritt b) 0,6 bis 1,0 Mol Thiolacton der Formel II gelöst in 0,8 bis 1,2 kg Tetrahydrofuran, bezogen auf 1 Mol in Schritt a) eingesetztes 1,4-Dichlorbutan, verwendet.
d) Verfahren, wobei man bei Schritt b) unter reduziertem Druck, vorzugsweise zwischen 0,1 und 0,3 bar, nachrührt.
e) Verfahren, wobei man Schritt c) bei einem Druck zwischen 0,5 und 1,0 bar durchführt, insbesondere wobei man das Kohlendioxid innerhalb von 15 bis 45 Minuten überleitet, und/oder wobei man das nach Überleiten des Kohlendioxids erhaltene Gemisch unter Druckausgleich auf Temperaturen zwischen 30 und 60 °C erwärmt.
f) Verfahren, wobei man Schritt d) bei Temperaturen zwischen 45° und 70 °C durchführt.
g) Verfahren, wobei man Schritt e) bei Temperaturen zwischen 30° und 55 °C durchführt.
h) Verfahren, wobei man bei Schritt f) einen aromatischen Kohlenwasserstoff wählt und die organische Phase mit einer wäßrigen Lauge wäscht, die einen pH-Wert zwischen 8,0 und 9,5, vorzugsweise zwischen 8,4 und 9,1, aufweist.

Ein weiterer Gegenstand der Erfindung ist ein Verfahren zur Herstellung von D-(+)-Biotin, dadurch gekennzeichnet, dass eine Verbindung der Formel I nach dem erfindungsgemäßen Verfahren hergestellt und anschließend in D-(+)-Biotin umgewandelt wird.

Die als Ausgangsmaterialien verwendete Verbindung der Formel II, sowie die erfindungsgemäß hergestellte Verbindung der Formel I sind bekannte Verbindungen (z.B. EP 0 084 377 R = Benzyl oder EP 0 273 270 R = 1-Phenylethyl).

Die nach der Dehydratisierung erhaltene Verbindung der Formel I ist, wie bereits erwähnt, eine bekannte Verbindung (z.B. EP-OS 084 377) und kann in bekannter Weise, d.h. durch Hydrierung der Doppelbindung und Abspaltung der Schutzgruppen an den Stickstoffatomen leicht in D-(+)-Biotin überführt werden (z.B. CH-PS 556867).

Die nachfolgenden Beispiele erläutern die Erfindung.

### Beispiel 1

Herstellung von cis-2-Oxo-1,3-dibenzyl-4-(4-carboxybutyl-1-iden)-hexahydro-1H-thieno[3,4-d]imidazol

Unter Stickstoff werden 41,5 g (1,69 Mol) Magnesiumspäne in 489,5 g THF vorgelegt und zum Sieden erhitzt. Zu dieser Suspension gibt man innerhalb von etwa 1 Stunde eine Lösung von 117,0 g (0,91 Mol) Dichlorbutan in 1246,0 g THF sowie 1,23 g (0,0065 Mol) Dibromethan. Die Reaktionsmischung wird 45 Minuten zum Sieden erhitzt und 16 Stunden bei Raumtemperatur nachgerührt.

Anschließend wird auf -15 °C abgekühlt. Eine Lösung von 222,2 g (0,65 Mol) (+)-cis-1,3-Dibenzyl-hexahydro-1H-thieno[3,4-d]imidazol-2,4-dion in 867,8 g THF wird innerhalb von 95 Minuten bei einer Temperatur zwischen -14 und -16 °C hinzugetropft.

Nach 10minütigem Rühren wird das Reaktionsgefäß bei einem Druck von 0,2 bar innerhalb von 5 Minuten evakuiert.

Bei einer Temperatur von -20 °C und einem Druck von 0,6 bar wird 15 Minuten Kohlendioxid übergeleitet wobei die Temperatur langsam auf 0 °C ansteigt.

Bei weiterer Kohlendioxid-Überleitung bei Temperaturen bis 15 °C stabilisiert sich der Druck bei 0,9 bar.

Das Reaktionsgemisch wird innerhalb von 40 Minuten unter Druckausgleich auf +50 °C erwärmt.

Anschließend fügt man 1000 g 30%ige Schwefelsäure folgendermaßen hinzu:
Innerhalb von 10 Minuten werden 150 ml Schwefelsäure zugefügt, wobei die Temperatur auf 60-62 °C steigt.
Innerhalb von 15 Minuten wird die restliche Schwefelsäure hinzugegeben, wobei die Temperatur auf 52 °C sinkt. Anschließend wird das Reaktionsgemisch 70 Minuten bei Temperaturen zwischen 52 und 56 °C gehalten und gerührt. Die Phasen werden bei dieser Temperatur separiert und die organische Phase im Vakuum eingeengt. Der Rückstand wird in 650 ml Toluol aufgenommen, die restliche Schwefelsäure wird abgetrennt und die organische Phase wird mit 250 ml Wasser gewaschen.

Anschließend wird die organische Phase mit 715 ml 1 molarer Natronlauge versetzt. Nach Trennen der Phasen wird die wäßrige Phase 2× mit 250 ml Toluol extrahiert. Die organische Phasen werden vereinigt und im Vakuum eingedampft. Die wäßrige Phase wird mit 650 ml Toluol versetzt, mit 45 ml konzentrierter Salzsäure auf pH 6,5 eingestellt. Die organische Phase wird abgetrennt und im Vakuum eingedampft. Man erhält 228,3 g (83,1 % d.Th.) des gewünschten Produktes in Form von weißen Nadeln mit einem Schmelzpunkt von 80-83,5 °C.

Ausgehend von 0,5 Mol (+)-cis-1,3-Di-(1-phenyl-ethyl)-hexahydro-1H-thieno[3,4-d]imidazo(-2,4-dion) erhält man analog 164,9 g (73,2 % d.Th.) cis-2-Oxo-1,3-di-(1-phenylethyl)-4-(4-carboxybutyl-1-iden)-hexahydro-1H-thieno[3,4-d]imidazol als gelbliches Öl.

## Patentansprüche

1. Verfahren zur Herstellung eines D-(+)-Biotin-Zwischenproduktes der Formel I, 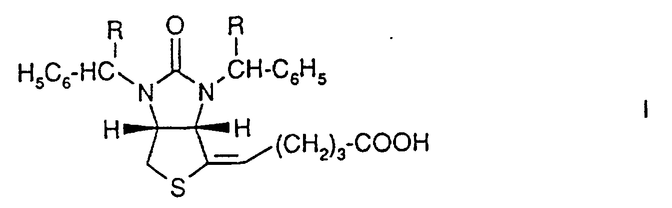 worin
R H oder C₁₋₆-Alkyl bedeutet,
durch Umsetzen des Thiolactons der Formel II, 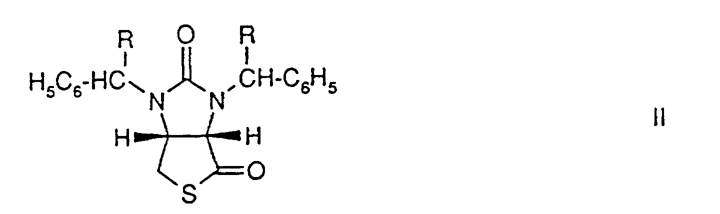 worin R die angegebene Bedeutung besitzt, mit 1,4-Dimagnesiumchlorbutan und Kohlendioxid in einem inerten Lösungsmittel und anschließende Wasserabspaltung mit einer Mineralsäure, **dadurch gekennzeichnet, daß** man nacheinander folgende Schritte durchführt:
a) Umsetzung von 1,4-Dichlorbutan mit Magnesium-Spänen in Gegenwart katalytischer Mengen von 1,2-Dibromethan in Tetrahydrofuran als Lösungsmittel,
b) Hinzufügen des Thiolactons der Formel II gelöst in Tetrahydrofuran bei Temperaturen zwischen -25° und -15 °C und gegebenenfalls Nachrühren,
c) Überleitung von CO₂ bei Temperaturen zwischen -25° und +15 °C,
d) Zugabe von Schwefelsäure, zur Neutralisierung und Dehydratisierung,
e) Separieren der Phasen und unter reduziertem Druck einengen,
f) Lösen des Rückstandes in einem Kohlenwasserstoff als Lösungsmittel und Waschen der organischen Phasen mit wäßriger Lauge,
g) Ansäuern der wäßrigen Phase mit einer Mineralsäure und Extraktion der wäßrigen Phase mit einem Kohlenwasserstoff,
h) Einengen der organischen Phase g) unter reduziertem Druck.

2. Verfahren nach Anspruch 1, **dadurch gekennzeichnet, daß** man Schritt a) bei Temperaturen zwischen 40 und 70 °C durchführt.

3. Verfahren nach Anspruch 1 oder 2, **dadurch gekennzeichnet, daß** man in Schritt a) 1,6 bis 2,2 Mol Magnesium, 0,005 bis 0,020 Mol 1,2-Dibromethan, und insgesamt 1,5 bis 2,2 kg Tetrahydrofuran bezogen auf 1 Mol 1,4-Dichlorbutan, einsetzt.

4. Verfahren nach einem der vorangehenden Ansprüche, **dadurch gekennzeichnet, daß** man in Schritt b) 0,6 bis 1,0 Mol Thiolacton der Formel II gelöst in 0,8 bis 1,2 kg Tetrahydrofuran, bezogen auf 1 Mol in Schritt a) eingesetztes 1,4-Dichlorbutan, verwendet.

5. Verfahren nach einem der vorangehenden Ansprüche, **dadurch gekennzeichnet, daß** man bei Schritt b) unter reduziertem Druck, vorzugsweise zwischen 0,1 und 0,3 bar, nachrührt.

6. Verfahren nach einem der vorangehenden Ansprüche, **dadurch gekennzeichnet, daß** man Schritt c) bei einem Druck zwischen 0,5 und 1,0 bar durchführt.

7. Verfahren nach Anspruch 6, **dadurch gekennzeichnet, daß** man das Kohlendioxid innerhalb von 15 bis 45 Minuten überleitet.

8. Verfahren nach Anspruch 6 oder 7, **dadurch gekennzeichnet, daß** man das nach Überleiten des Kohlendioxids erhaltene Gemisch unter Druckausgleich auf Temperaturen zwischen 30 und 60 °C erwärmt.

9. Verfahren nach einem der vorangehenden Ansprüche, **dadurch gekennzeichnet, daß** man Schritt d) bei Temperaturen zwischen 45° und 70 °C durchführt.

10. Verfahren nach einem der vorangehenden Ansprüche, **dadurch gekennzeichnet, daß** man Schritt e) bei Temperaturen zwischen 30° und 55 °C durchführt.

11. Verfahren nach einem der vorangehenden Ansprüche, **dadurch gekennzeichnet, daß** man bei Schritt f) einen aromatischen Kohlenwasserstoff wählt und die organische Phase mit einer wäßrigen Lauge wäscht, die einen pH-Wert zwischen 8,0 und 9,5, vorzugsweise zwischen 8,4 und 9,1, aufweist.

12. Verfahren zur Herstellung von D(+)-Biotin, **dadurch gekennzeichnet, dass** eine Verbindung der Formel I nach mindestens einem der Ansprüche 1-11 hergestellt und anschließend in D-(+)-Biotin umgewandelt wird.

## Claims

1. Process for the preparation of a D-(+)-biotin intermediate of the formula I 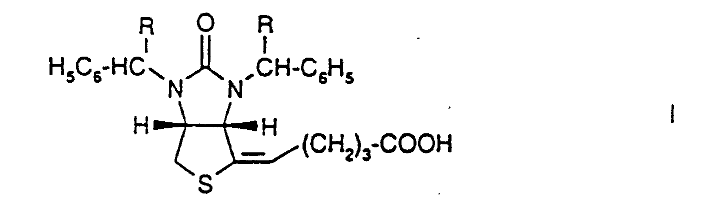 in which
R is H or C₁₋₆-alkyl,
by reacting the thiolactone of the formula II 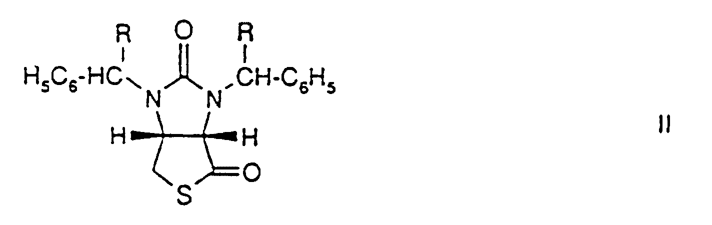 in which R has the meaning indicated, with 1,4-dimagnesiumchlorobutane and carbon dioxide in an inert solvent and subsequent elimination of water using a mineral acid, **characterized in that** the following steps are carried out successively:
a) reaction of 1,4-dichlorobutane with magnesium turnings in the presence of catalytic amounts of 1,2-dibromoethane in tetrahydrofuran as a solvent,
b) adding the thiolactone of the formula II, dissolved in tetrahydrofuran at temperatures between -25° and -15°C, and, if appropriate, subsequent stirring,
c) passing over CO₂ at temperatures between -25° and +15°C,
d) addition of sulphuric acid for neutralization and dehydration,
e) separating the phases and concentrating under reduced pressure,
f) dissolving the residue in a hydrocarbon as a solvent and washing the organic phases with aqueous alkali,
g) acidifying the aqueous phase with a mineral acid and extraction of the aqueous phase with a hydrocarbon,
h) concentrating the organic phase of g) under reduced pressure.

2. Process according to Claim 1, **characterized in that** step a) is carried out at temperatures between 40 and 70°C.

3. Process according to Claim 1 or 2, **characterized in that**, in step a), 1.6 to 2.2 mol of magnesium, 0.005 to 0.020 mol of 1,2-dibromoethane, and a total of 1.5 to 2.2 kg of tetrahydrofuran are employed relative to 1 mol of 1,4-dichlorobutane.

4. Process according to one of the preceding claims, **characterized in that**, in step b), 0.6 to 1.0 mol of thiolactone of the formula II dissolved in 0.8 to 1.2 kg of tetrahydrofuran are used, relative to 1 mol of 1,4-dichlorobutane employed in step a).

5. Process according to one of the preceding claims, **characterized in that**, in step b), subsequent stirring is carried out under reduced pressure, preferably between 0.1 and 0.3 bar.

6. Process according to one of the preceding claims, **characterized in that** step c) is carried out at a pressure between 0.5 and 1.0 bar.

7. Process according to Claim 6, **characterized in that** the carbon dioxide is passed over in the course of 15 to 45 minutes.

8. Process according to Claim 6 or 7, **characterized in that** the mixture obtained after passing over the carbon dioxide is warmed to temperatures between 30 and 60°C with pressure equalization.

9. Process according to one of the preceding claims, **characterized in that** step d) is carried out at temperatures between 45° and 70°C.

10. Process according to one of the preceding claims, **characterized in that** step e) is carried out at temperatures between 30° and 55°C.

11. Process according to one of the preceding claims, **characterized in that**, in step f), an aromatic hydrocarbon is selected and the organic phase is washed with an aqueous alkali which has a pH between 8.0 and 9.5, preferably between 8.4 and 9.1.

12. Process for the preparation of D-(+)-biotin, **characterized in that** a compound of the formula I according to at least one of Claims 1-11 is prepared and then converted into D-(+)-biotin.

## Revendications

1. Procédé pour la préparation d'un produit intermédiaire pour la synthèse de la D-(+)-biotine, de formule I 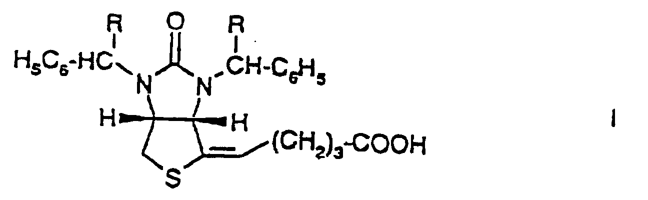 dans laquelle
R représente H ou un groupe alkyle en C₁-C₆,
par mise en réaction de la thiolactone de formule II 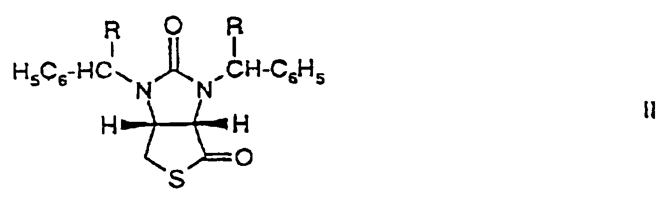 dans laquelle R a la signification indiquée, avec du 1,4-dimagnésium-chlorobutane et du dioxyde de carbone dans un solvant inerte et déshydratation subséquente avec un acide minéral, **caractérisé en ce qu'**on effectue successivement les étapes suivantes :
a) réaction du 1,4-dichlorobutane avec des tournures de magnésium en présence de quantités catalytiques de 1,2-dibromoéthane dans du tétrahydrofuranne en tant que solvant,
b) addition de la thiolactone de formule II, dissoute dans du tétrahydrofuranne, à des températures comprises entre -25°C et-45°C, et éventuellement agitation subséquente,
c) introduction de CO₂ à des températures comprises entre -25°C et +15°C,
d) addition d'acide sulfurique pour la neutralisation et la déshydratation,
e) séparation des phases et concentration sous pression réduite,
f) dissolution du résidu dans un hydrocarbure en tant que solvant et lavage des phases organiques avec une solution aqueuse d'un hydroxyde de métal alcalin,
g) acidification de la phase aqueuse avec un acide minéral et extraction de la phase aqueuse avec un hydrocarbure,
h) concentration de la phase organique g) sous pression réduite.

2. Procédé selon la revendication 1, **caractérisé en ce qu'**on effectue l'étape a) à des températures comprises entre 40 et 70°C.

3. Procédé selon la revendication 1 ou 2, **caractérisé en ce qu'**on utilise dans l'étape a) 1,6 à 2,2 moles de magnésium, 0,005 à 0,020 mole de 1,2-dibromoéthane et au total 1,5 à 2,2 kg de tétrahydrofuranne, par rapport à 1 mole de 1,4-dichlorobutane.

4. Procédé selon l'une quelconque des revendications précédentes, **caractérisé en ce qu'**on utilise dans l'étape b) 0,6 à 1,0 mole de thiolactone de formule II, dissoute dans 0,8 à 1,2 kg de tétrahydrofuranne, par rapport à 1 mole de 1,4-dichlorobutane utilisé dans l'étape a).

5. Procédé selon l'une quelconque des revendications précédentes, **caractérisé en ce qu'**on effectue l'agitation subséquente dans l'étape b) sous une pression réduite, de préférence entre 0,1 et 0,3 bar.

6. Procédé selon l'une quelconque des revendications précédentes, **caractérisé en ce qu'**on effectue l'étape c) sous une pression comprise entre 0,5 et 1,0 bar.

7. Procédé selon la revendication 6, **caractérisé en ce qu'**on fait passer le dioxyde de carbone pendant 15 à 45 minutes.

8. Procédé selon la revendications 6 ou 7, **caractérisé en ce qu'**après, avoir fait passer le dioxyde de carbone on chauffe le mélange obtenu, sous équilibrage de pression, à des températures comprises entre 30 et 60°C.

9. Procédé selon l'une quelconque des revendications précédentes, **caractérisé en ce qu'**on effectue l'étape d) à des températures comprises entre 45 et 70°C.

10. Procédé selon l'une quelconque des revendications précédentes, **caractérisé en ce qu'**on effectue l'étape e) à des températures comprises entre 30 et 55°C.

11. Procédé selon l'une quelconque des revendications précédentes, **caractérisé en ce que** dans l'étape f) on choisit un hydrocarbure aromatique et on lave la phase organique avec une solution aqueuse qui présente un pH compris entre 8,0 et 9,5, de préférence entre 8,4 et 9,1.

12. Procédé pour la préparation de la D(+)-biotine, **caractérisé en ce qu'**on prépare un composé de formule I selon au moins l'une des revendications 1 à 11 et on le convertit ensuite en D-(+)-biotine.
